# EUROPEAN PATENT APPLICATION

(11) **EP 1 439 083 A2**
(43) Date of publication of application: **21.07.2004**
(21) Application number: 04100129.8
(22) Date of filing: 16.01.2004
(51) Int. Cl.: B60H 3/00

(54) **Vehicle air conditioning and ventilation system equipped with an automatic controlled air freshening device**

(30) Priority: 17.01.2003 IT TO20030023
(71) Applicant: C.R.F. Società Consortile per Azioni, 10043 Orbassano (Torino) (IT)
(72) Inventor: Malvicino, Carloandrea, 10146 Torino (IT); Marzorati, Diego, 10136 Torino (IT); Bonino, Francesco, 12084 Mondovi (IT); Fino, Federica, c/o C.R.F. Societa per Azioni, 10043 Orbassano (IT)
(74) Representative: Jorio, Paolo, Dr. Ing.

(57) **Abstract**

There is described an air conditioning and ventilation system (1) for a vehicle, equipped with an automatic controlled air freshening device (3), which has a perfume injecting device (11) for injecting one or more perfumes into the passenger compartment (2) of the vehicle, and an electronic central control unit (12) for controlling the perfume injecting device (11) to regulate perfume injection.

## Description

The present invention relates to a vehicle air conditioning and ventilation system equipped with an automatic controlled air freshening device.

As is known, vehicle passenger compartments are currently freshened using air fresheners or deodorants located inside the passenger compartment of the vehicle to dispense a given perfume to conceal, correct, or eliminate any unpleasant odours and so enhance the well-being of the occupants.

Some of the most widely used air fresheners comprise a dispenser made of porous material, e.g. paper or similar, which is impregnated with a given perfume, and which, when placed in direct contact with the air, gradually releases the perfume in uncontrolled manner until it runs out.

Other types of air fresheners comprise a plastic container closed partly by a porous membrane and containing a liquid substance mixed with a given perfume. The container is normally fitted to the dashpanel of the vehicle, at the air inlet, where the in-coming air flowing over the membrane closing the container gradually "draws" the perfume into, and so freshens, the passenger compartment. In this case, perfume is dispensed solely from the specific air inlet to which the container is fitted, as opposed to involving the passenger compartment ventilation system as a whole.

Both the above types of air freshener have the major drawback of dispensing perfume inside the passenger compartment of the vehicle in uncontrolled, irregular manner, so that effectiveness varies depending on depletion of the perfumed substance in the freshener. In the case in question, diffusion of the perfume within the passenger compartment decreases gradually as the substance in the freshener is used up, and until it runs out completely, thus resulting, in some cases, in unpleasant odours for which a tolerance is gradually acquired.

It is an object of the present invention to provide a vehicle air conditioning and ventilation system equipped with an air freshening device designed to automatically dispense perfume inside the passenger compartment of the vehicle in controlled manner, so that the perfume concentration inside the passenger compartment remains constant at all times.

According to the present invention, there is provided an air conditioning and ventilation system for a vehicle, characterized by comprising a device for automatically dispensing perfume inside the passenger compartment of the vehicle in controlled manner, and comprising perfume injecting means for injecting one or more perfumes inside the passenger compartment of the vehicle, and electronic control means for controlling said perfume injecting means to regulate perfume injection.

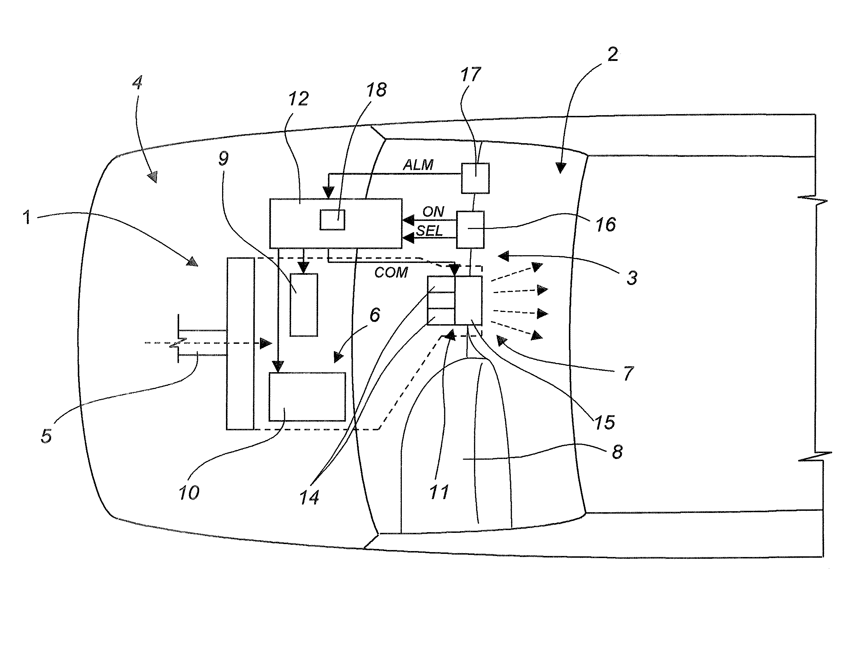

A non-limiting embodiment of the invention will be described by way of example with reference to the accompanying drawing, which shows, schematically, a vehicle air conditioning and ventilation system equipped with a device for automatically dispensing perfume inside the passenger compartment of the vehicle, in accordance with the teachings of the present invention.

Number 1 in the accompanying drawing indicates as a whole an air conditioning and ventilation system of a vehicle passenger compartment 2, equipped with an automatic air freshening device 3 designed to fully automatically regulate the distribution of one or more perfumes inside passenger compartment 2 of the vehicle.

Air conditioning and ventilation system 1 is housed inside the engine compartment 4 of the vehicle, and comprises: an air supply circuit 5 (shown partly in the accompanying drawing) for feeding air into passenger compartment 2 of the vehicle and defined, for example, by a circuit for recirculating the air inside passenger compartment 2 and/or drawing in outside air; and an air conditioning assembly 6 connected to supply circuit 5 to cool and/or heat, on command, the air fed into passenger compartment 2.

More specifically, in the example shown in the accompanying drawing, air conditioning assembly 6 is located at the end portion of supply circuit 5, e.g. at an outlet 7 formed on the dashpanel 8 of the vehicle, and comprises an evaporator or radiator 9 for withdrawing heat from the air and yielding it via a condensing member (not shown) outside passenger compartment 2; and a heating device 10 for heating the air before it is fed into passenger compartment 2. Air conditioning assembly 6 is of known type, and therefore not described further.

Automatic air freshening device 3, on the other hand, comprises a perfume injecting device 11 connected to supply circuit 5 and for spraying or injecting one or more perfumes into the air circulating inside supply circuit 5; and an electronic central control unit 12 for controlling operation of perfume injecting device 11 so as to selectively control injection of each perfume into the air and the amount of perfume ejected.

More specifically, perfume injecting device 11 comprises a number of perfume tanks 14, each containing a respective perfume; and an ejector assembly 15 connected to perfume tanks 14 and having a number of nozzles (not shown) by which the perfume in tanks 14 is ejected. In the example shown, perfume injecting device 11 may be defined by at least one cartridge having an injection head, and identical with disposable ink cartridges of "Bubble Ink Jet" printers. In this case, each cartridge obviously comprises an injection head defining ejector assembly 15, and a tank containing perfume as opposed to ink. The cartridges may be housed inside a seat (not shown) formed on dashpanel 8, at outlet 7, to enable fast, easy replacement of the cartridges.

In connection with the above, it should be pointed out that perfume injecting device 11 is connected to supply circuit 5 to emit perfume through one or more outlets 7 (only one of which is shown in the drawing).

Electronic central control unit 12 supplies perfume injecting device 11 with a control signal COM containing coded information indicating the type and quantity of perfume to be injected. The control signal COM supplied by electronic central control unit 12 may also contain a coded time interval indicating the injection duration of each perfume.

In other words, by means of control signal COM, electronic central control unit 12 controls perfume injecting device 11 so as to automatically regulate, for each perfume, both the quantity injected into the air and its injection time interval.

The perfume-regulating information coded by electronic central control unit 12 in signal COM may be processed by electronic central control unit 12 on the basis of a number of perfume selection signals SEL entered directly by the vehicle user on a control panel 16 located inside passenger compartment 2 and communicating with electronic central control unit 12.

More specifically, control panel 16 may be defined, preferably though not necessarily, by an on-vehicle computer located on dashpanel 8, and which may comprise a control keyboard (not shown) enabling any occupant of the vehicle to select the quantity and type of perfume/s desired and the injection time interval of each perfume; and, preferably though not necessarily, a display (not shown) for displaying, for example, the various types of perfumes selectable; the degree of fragrance, i.e. the quantity of perfume injected, to regulate the perfume strength inside the passenger compartment as required; the amount of perfume remaining inside the respective perfume tank 14; and the injection intervals of each perfume.

In addition to the above functions of automatically regulating air freshening of passenger compartment 2, to conceal, correct or eliminate any unpleasant odours, automatic air freshening device 3 may also be used as a vehicle-to-driver olfactory communication channel, to communicate information which can only be perceived by the driver's sense of smell, and indicating, for example, a sudden hazard condition. For which purpose, automatic air freshening device 3 may preferably, though not necessarily, comprise a driver-status detecting device 17, which supplies electronic central control unit 12 with an alarm signal ALM indicating a fatigue condition of the driver. Electronic central control unit 12 therefore commands perfume injecting device 11 to inject a predetermined quantity of a given perfume to heighten the level of awareness of the driver, if overcome by drowsiness. More specifically, driver-status detecting device 17 may provide for determining driver fatigue, and generating the alarm signal ALM, on the basis of a number of intrinsic vehicle parameters and the way in which the driver is driving the vehicle.

In connection with the above, it should be pointed out that the functions performed by electronic central control unit 12 may be implemented by the vehicle electronic central control unit, which, in addition to perfume injecting device 11, can also control air conditioning and ventilation system 1, and receives an input signal ON indicating activation of air conditioning and ventilation system 1. The ON signal may be supplied directly from control panel 16 or by any other on-vehicle device for regulating air conditioning and ventilation system 1 and by which air conditioning and ventilation system 1 can be turned on/off by the occupants of the vehicle.

In the example shown, perfume injecting device 11 may preferably, though not necessarily, be activated after air conditioning and ventilation system 1 is turned on, so that the air circulating in the supply circuit "draws" into passenger compartment 2 the drops of perfume injected by perfume injecting device 11. In other words, activation of perfume injecting device 11 may be dependent on activation of air conditioning and ventilation system 1, which may be controlled by the driver from control panel 16.

Electronic central control unit 12 may also comprise a self-teach device 18, which acquires and memorizes, each time, the information relating to the perfume selected by a given driver, such as the type and quantity of preferred perfumes and the injection intervals of each, so that, once the driver is identified, e.g. by means of an identity card, automatic air freshening device 3 may operate on the basis of stored, self-taught preferences to advantageously freshen the passenger compartment according to the personal preference of each driver.

Operation of automatic air freshening device 3 is easily deducible from the foregoing description with no further explanation required.

The advantages of the automatic air freshening device according to the present invention will be clear from the foregoing description. In particular, in addition to controlled, constant perfume distribution, regardless of how much is left in the tanks, the device also allows the perfume strength, quantity, and time interval to be selected as required, thus improving olfactory conditions inside the passenger compartment of the vehicle.

Freshening of the passenger compartment may be personalized either by selecting any one of the perfumes in the cartridge, or, given the ease with which the perfume injecting device is replaced, by selecting perfume cartridges other than the one installed.

Finally, as stated, the automatic air freshening device has the big advantage of being usable as an olfactory communication channel to warn the driver of any critical condition caused by lack of attention resulting, for example, from drowsiness.

Clearly, changes may be made to the automatic air freshening device as described and illustrated herein without, however, departing from the scope of the present invention as defined in the accompanying Claims.

## Claims

1. An air conditioning and ventilation system (1) for a vehicle, **characterized by** comprising a device for automatically dispensing perfume inside the passenger compartment (2) of the vehicle in controlled manner, and comprising perfume injecting means (11) for injecting one or more perfumes inside the passenger compartment (2) of the vehicle, and electronic control means (12) for controlling said perfume injecting means (11) to regulate perfume injection.

2. An air conditioning and ventilation system as claimed in Claim 1, **characterized in that** said electronic control means (12) regulate the quantity and/or injection time interval of each perfume injected by said perfume injecting means (11).

3. An air conditioning and ventilation system as claimed in Claim 1 or 2, **characterized by** comprising selection means (16) for supplying said electronic control means (12) with first signals (SEL) containing coded information indicating the perfume setting made by the user; said electronic control means (12) controlling said perfume injecting means (11) as a function of said first signals (SEL).

4. An air conditioning and ventilation system as claimed in any one of the foregoing Claims, **characterized by** comprising an air supply circuit (5) for feeding air into said passenger compartment (2); said perfume injecting means (11) being located in said air supply circuit (5).

5. An air conditioning and ventilation system as claimed in Claim 4, **characterized in that** said air supply circuit (5) comprises at least one outlet (7) by which air is fed into said passenger compartment (2); said perfume injecting means (11) being located at said at least one outlet (7).

6. An air conditioning and ventilation system as claimed in Claim 4, **characterized in that** said air supply circuit (5) comprises a number of outlets (7) by which air is fed into said passenger compartment (2); said perfume injecting means (11) being so located in said air supply circuit (5) as to feed perfume into said passenger compartment (2) through all the outlets (7).

7. An air conditioning and ventilation system as claimed in any one of the foregoing Claims, **characterized in that** said perfume injecting means (11) comprise at least one cartridge having an injection head and similar to a cartridge of an ink-jet printer.

8. An air conditioning and ventilation system as claimed in any one of the foregoing Claims, **characterized in that** said electronic control means (12) control said perfume injecting means (11) as a function of an alarm signal (ALM) indicating fatigue of the driver of the vehicle.

9. An air conditioning and ventilation system as claimed in Claim 8, **characterized by** comprising detecting means (17) for supplying said alarm signal (ALM) indicating fatigue of the driver of the vehicle.

10. An air conditioning and ventilation system as claimed in any one of the foregoing Claims, **characterized in that** said electronic control means (12) comprise a self-teach unit (18), which acquires and stores information relating to the perfume selected by a given driver, so as to control said perfume injecting means (11), subsequent to the self-teaching stage, to dispense perfume in accordance with the stored, self-taught information.
